# EUROPEAN PATENT SPECIFICATION

(11) **EP 3 793 580 B1**
(45) Date of publication and mention of the grant of the patent: **31.07.2024**
(21) Application number: 19730543.6
(22) Date of filing: 14.05.2019
(51) Int. Cl.: A61K 35/747, C12R 1/225, A23L 33/135

(54) **PROBIOTIC STRAIN LACTOBACILLUS KEFIRI SGL 13 AND USE THEREOF FOR THE PREVENTION AND TREATMENT OF INTESTINAL INFLAMMATION AND IN THE PREVENTION OF INTESTINAL NEOPLASMS**
PROBIOTISCHER STAMM LACTOBACILLUS KEFIRI SGL 13 UND SEINE VERWENDUNG ZUR VORBEUGUNG UND BEHANDLUNG VON DARMENTZÜNDUNG UND ZUR VORBEUGUNG VON DARMNEOPLASMEN
SOUCHE PROBIOTIQUE DE LACTOBACILLUS KEFIRI SGL 13 ET SON UTILISATION POUR LA PRÉVENTION ET LE TRAITEMENT DE L'INFLAMMATION INTESTINALE ET DANS LA PRÉVENTION DES NÉOPLASMES INTESTINAUX

(30) Priority: 14.05.2018 IT 201800005359
(43) Date of publication of application: 24.03.2021
(73) Proprietor: Nutraceutical Technology Pharma Biotech S.r.l., 64020 Castellalto (TE) (IT)
(72) Inventor: MARINI, Umberto, 64020 Castelnuovo Vomano (IT)
(74) Representative: Zaccaro, Elisabetta
(86) International application number: PCT/IB2019/053971
(87) International publication number: WO 2019/220329

(56) References cited:
- WO-A2-2017/137920
- CN-A- 104 560 793
- YONGCHEN ZHENG ET AL: "Probiotic Properties of Lactobacillus Strains Isolated from Tibetan Kefir Grains. article e69868", PLOS ONE, vol. 8, no. 7, 22 July 2013 (2013-07-22), US, pages 1 - 8, XP055325057, ISSN: 1932-6203, DOI: 10.1371/journal.pone.0069868
- P. CARASI ET AL: "Impact of Kefir Derived Lactobacillus kefiri on the Mucosal Immune Response and Gut Microbiota", JOURNAL OF IMMUNOLOGY RESEARCH, vol. 137, no. 3, 1 January 2015 (2015-01-01), US, pages 781 - 12, XP055324639, ISSN: 2314-8861, DOI: 10.1155/2015/361604
- L. ZAVALA ET AL: "Selected Lactobacillus strains isolated from sugary and milk kefir reduce Salmonella infection of epithelial cells in vitro", BENEFICIAL MICROBES, vol. 7, no. 4, 1 September 2016 (2016-09-01), NL, pages 585 - 595, XP055513201, ISSN: 1876-2883, DOI: 10.3920/BM2015.0196
- YU-TANG TUNG ET AL: "Therapeutic Potential of Andrographolide Isolated from the Leaves of Andrographis paniculata Nees for Treating Lung Adenocarcinomas", EVIDENCE-BASED COMPLEMENTARY AND ALTERNATIVE MEDICINE, vol. 2013, 1 January 2013 (2013-01-01), pages 1 - 8, XP055513460, ISSN: 1741-427X, DOI: 10.1155/2013/305898
- PAULA CARASI ET AL: "Adhesion properties of potentially probiotic Lactobacillus kefiri to gastrointestinal mucus", JOURNAL OF DAIRY RESEARCH, vol. 81, no. 01, 29 October 2013 (2013-10-29), GB, pages 16 - 23, XP055325052, ISSN: 0022-0299, DOI: 10.1017/S0022029913000526
- BOLLA PA: "Kefir-isolated bacteria and yeasts inhibit Shigella flexneri invasion and modulate pro-inflammatory response on intestinal epithelial cells", vol. 7, no. 1, 1 February 2016 (2016-02-01), pages 103 - 110, XP009507253, ISSN: 1876-2883, Retrieved from the Internet <URL:https://www.ncbi.nlm.nih.gov/pubmed/26503738> DOI: 10.3920/BM2015.0061
- FEDERICA FEDERICI ET AL: "Draft genome sequence of Lactobacillus kefiri SGL 13, a potential probiotic strain isolated from Kefir grains", MICROBIOLOGY RESOURCE ANNOUNCEMENTS, vol. 7, no. 4, 2 August 2018 (2018-08-02), pages 1 - 2, XP055605429, DOI: 10.1128/MRA.00937-18

## Description

### FIELD OF THE INVENTION

The present invention relates to the probiotic strain *Lactobacillus kefiri SGL* 13 and use thereof, alone or in association with prebiotics and plant extracts, for making a food and pharmaceutical composition for use as supplement, medical or pharmaceutical device. The use of the probiotic strain *Lactobacillus kefiri SGL* 13 in the prevention and treatment of intestinal inflammation is also described.

### STATE OF THE ART

Interactions between commensal bacteria, intestinal epithelial cells and immune cells play a crucial role in maintaining intestinal homeostasis. Microbial recognition through specific receptors induces the expression and release of various immune mediators, such as chemokines and pro- or anti-inflammatory cytokines that contribute to orchestrating both the innate and adaptive immune responses. The use of probiotics to modulate immune responses at the mucosal and systemic level is today a very interesting alternative regarding prevention and treatment of infectious diseases of various immunopathies, such as inflammatory bowel disease (IBD) and allergies, or even metabolic disorders.

Important immunomodulatory and anti-inflammatory properties are attributable to a fermented product known as Kefir.

Kefir granules represent a complex composition of microbial species such as the predominance of lactic acid bacteria, acetic bacteria, yeasts and fungi. These granules, if used to ferment milk, produce a healthy drink called "Kefir" that has been used for centuries in Caucasian countries and nowadays is spread all over the world, with healthy effects on populations using it regularly. In fact, several beneficial properties, such as immunostimulant, antimicrobial, anti-inflammatory and anticancer, have been associated with the consumption of kefir. Therefore, the study of probiotic properties of microorganisms isolated from kefir constitutes a field of great interest for the development of functional foods.

Microbial species present in kefir granules include: homofermentative and heterofermentative lactic bacteria, and lactose fermenting and non-fermenting yeasts. *Lactobacillus paracasei ssp. paracasei, Lactobacillus acidophilus, Lactobacillus delbrueckii ssp. bulgaricus, Lactobacillus plantarum* and *L*. *kefiranofaciens* are the predominant species in kefir granules. However, these species represent only 20% of the lactobacilli in the final fermented drink, since 80% is comprised of *Lactobacillus kefiri.*

Several studies report the immunomodulatory and anti-inflammatory properties of *L. kefiri.* The *L. kefiri* IM002 strain is able to attenuate the pro-inflammatory response induced by *Salmonella Typhimurium* in intestinal epithelial cells HT-29, by reducing the secretion of IL-8, one of the main mediators of the inflammatory response.

A more recent *in vivo* study demonstrated that daily administration of a *L. kefiri* strain in the mouse for 21 days is related to an increase in secretory IgAs at fecal level, which is a key element in maintaining intestinal homeostasis and in the protection of the intestinal mucosa from pathogens. Furthermore, already after a week of administration there is a reduction of the pro-inflammatory cytokines IL-1β and IL-17A at the ileum level. After 21 days, there is a reduction of the cytokines IL-6, GM-CSF, IL-17A and IFN-γ at the level of the Peyer plaques, of IL-6, GM-CSF and IL-17A at the level of mesenteric lymph nodes, and of GM-CSF, IFN-γ and IL-1β in the colon.

Still at the ileum level, the treated animals show an increase in the levels of anti-inflammatory IL-10, as well as of the chemokine CXCL-1 (functionally analogous to human IL-8) involved in the recruitment of neutrophils from the lamina propria to the epithelium, and essential in the protection from dextran sodium sulfate (DSS) induced colitis.

Furthermore, WO2017/137920 describes a *L.kefiri* strain which adheres to and protects enterocytes from cytokine-induced inflammation.

In addition to regulation of immune and inflammatory response, *L. kefiri,* like many other lactobacilli, is able to prevent invasion by some enteropathogens at the intestinal level, on the one hand exploiting the adhesion properties to the epithelium, thus reducing the binding sites for pathogens, and on the other hand through an aggregation mechanism with the pathogens themselves. Adhesion to the mucosa lining the intestinal tract is the first step required for a probiotic to interact with host cells and trigger a particular response.

The exopolysaccharides (EPS) produced by lactic ferments, and in particular by those species originating kefir granules, have a crucial role not only for fermented products (wherein they determine consistency, texture and rheological properties) but also in biofilm prevention processes, antioxidant activity, anticancer, and immunomodulatory properties. EPS are long saccharide chains whose main components are monosaccharides, such as glucose, galactose, fructose, and mannose. To date, the mechanisms by which these molecules implement their beneficial properties are still unclear, but there are numerous evidences that part of probiotic effects of lactic bacteria are attributable to surface bioactive compounds, such as EPS.

In the adhesion and aggregation mechanisms, a crucial role is also played by other surface molecules, i.e. proteins known as S-layers. The presence of S-layer proteins in *L. kefiri* has been demonstrated. Several studies demonstrate that an involvement of such proteins is at the basis of probiotic properties of some *L. kefiri* strains. In particular, they are able to prevent *Salmonella enterica* invasion of Caco-2 cells and inhibit the cytotoxic effect of *Clostridium difficile* toxins on Vero cells.

In some *L. kefiri* strains, removal of S-layers by extraction with lithium chloride and sodium hydroxide significantly reduces the adhesive properties of such strains to the gastrointestinal mucosa.

Recent studies have shown that the S-layers of a *L. kefiri* strain also have immunomodulatory properties as they are able to increase the secretion of the cytokines IL-6 and IL-10 by murine macrophages cell lines stimulated with *E*. *coli* LPS, compared to cells treated only with LPS.

In recent years, the importance of studying the amino acid sequence of S-layers in order to differentiate the *L. kefiri* strains and to better understand the mechanisms involved in the functionality of these proteins has emerged. In fact, the variability in the sequence of genes coding for S-layers, in particular at the C-terminal level, where the main sequence differences among the various strains are found, may be exploited precisely to distinguish the various strains and identify which are the protein domains mainly responsible for adhesion and aggregation properties.

Another mechanism by which probiotics, including *L. kefiri,* are able to regulate inflammatory processes is the production of short chain fatty acids (SCFAs). (19). Among the SCFAs produced, the most studied are acetic acid, propionic acid and butyric acid. In general, SCFAs, such as propionate and butyrate, inhibit the stimulus-induced expression of adhesion molecules, chemokine production, and consequently suppress monocyte/macrophage and neutrophil recruitment, suggesting an anti-inflammatory action. The amount and type of SCFAs produced at the intestinal level are related to the development of numerous diseases, including inflammatory bowel disease (IBD), in particular a lowering of butyric acid level in the colonocytes has been suggested to contribute to the genesis of ulcerative colitis.

Regarding the antitumor properties, it has been demonstrated that a strain of *L*. *kefiri* is able to induce apoptosis in human gastric adenocarcinoma (AGS) cells, in a dosedependent manner, without interfering with the peripheral blood mononuclear cells. In AGS cells treated with *L. kefiri* there is a reduced expression of Bcl-2 and a decrease in the mitochondrial membrane potential with consequent release of proapoptotic molecules causing the activation of caspases and eventually leading to apoptosis.

Similar effects have also been demonstrated on human myeloid leukemia cell lines (HL60/AR).

All these data together support the role of *L. kefiri* in the modulation of immune and inflammatory responses at the level of the intestinal mucosa, and also at a systemic level, and promote the use thereof for the prevention and treatment of intestinal diseases as well as other diseases.

In recent years, the beneficial effects of some plant extracts such as *Andrographis paniculata* with antimicrobial, anti-inflammatory and immunostimulant properties have also emerged in this field. Andrographolide is the major active component of this phytocomplex, and it is present mainly in the bush leaves. It is a diterpene lactone traditionally used in northern Europe for treatment and prophylaxis of colds. Despite secondary activities as antibacterial, antiviral, immunostimulant, hepatoprotectant and anti-carcinogen have been widely documented, its main function remains the anti-inflammatory one. Andrographolide prevents the activation of various inflammatory signaling pathways, such as the MAPK/ERK TLR3-dependent or the JAK/STAT stimulated by the IPN-β. The synthesis of COX-2, as well as prostaglandins (specifically, PGE2), the activation of the Nf-Kb transcription factor, the synthesis of IFN-β and pro-inflammatory cytokines are thus inhibited. Following a reduced maturation of dendritic cells, this diterpene prevents the activation and maturation of T cells, followed by a further decrease in inflammatory interleukins, such as IL-2 and IL-6, macrophages and IFN-γ in the inflammation source. Therefore, following treatment of murine cells with andrografolide, a general reduction in inflammation factors, i-NOS, PGE2, TNF-α, interferons and interleukins, was observed. The synergistic association between probiotics and plant extracts could represent an effective and safe strategy to enhance the beneficial effects of both.

In order to obtain an effective advantage with the intake of kefir drink, in such a way as to allow the modulation of immune responses at the level of the intestinal mucosa and at a systemic level, the intake of high amounts of the same kefir is necessary. Kefir has a sour taste, and from the caloric point of view it has the disadvantage that being necessary an intake of several times a day to get a benefit, it can cause an unwanted weight gain. In fact, kefir contains very variable amounts of fat, depending on the type of kefir, and mainly saturated fats. Finally, to avoid the not always pleasant taste, the intake of often caloric sugars and flavoring is required. Furthermore, the uncontrolled fermentation of kefir granules does not guarantee standardization of the yeast titer and the species represented.

The object of the present invention is therefore to provide an alternative to kefir drink, which has the same advantages from the health benefits point of view, but which does not have the known disadvantages of the product, guaranteeing a correct taxonomic definition of the yeasts, a correct titer and standardization of intake.

### SUMMARY OF THE INVENTION

The object of the present invention is to provide a bacterial strain belonging to the *Lactobacillus kefiri* species, wherein said strain is *L. kefiri* SGL 13 deposited with accession number DSM 27331 on 13/06/2013 at the International Depositary Authority Leibniz-Institut DSMZ - Deutsche Sammlung von Mikroorganismen und Zellkulturen GmbH. The *L. kefiri* SGL 13 DSM 27331, strain was deposited in the name of MD'E srl, via Nazionale 339, Castelnuovo Vomano (TE) 64020 Italy, which together with Sintal Dietetics is part of the same network of companies.

In a further aspect, the invention concerns a composition comprising the bacterial strain *L. kefiri* SGL 13, optionally added with pharmacologically acceptable excipients.

The invention further concerns a composition comprising the bacterial strain *L. kefiri* SGL 13, and a plant extract of *Andrographis paniculata* and/or one or more prebiotics, optionally added with pharmacologically acceptable excipients.

In a further aspect, the invention relates to the composition comprising the bacterial strain *L. kefiri* SGL 13, for use as a medicament.

In particular, said medicament is for use in the treatment and prevention of intestinal inflammations.

### DESCRIPTION OF THE FIGURES

The invention will now be described in detail, and with reference to the attached Figures wherein:

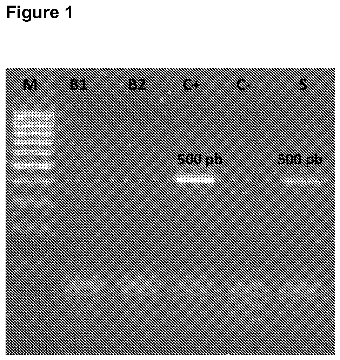

Figure 1 shows a photograph of an agarose gel in which an amplification is seen with species-specific primers: B1 and B2 (blanks, DNA-free samples to verify the absence of contamination in the PCR mix), C + and C- (positive control and negative control with respect to the amplified DNA), S (amplified sample corresponding to *L*. *kefiri* SGL 13 for appearance of the 500 pb band to confirm the species, M (molecular weight marker).

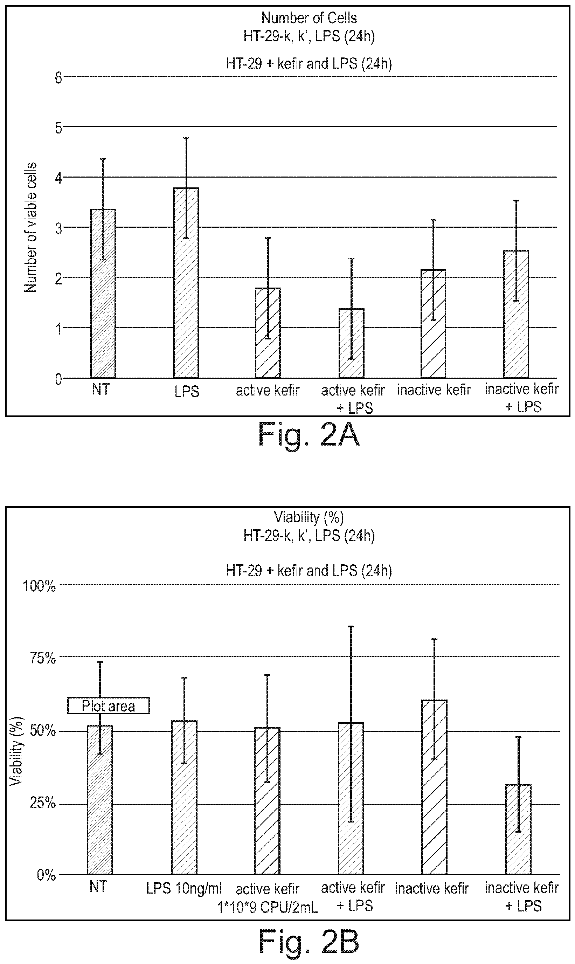

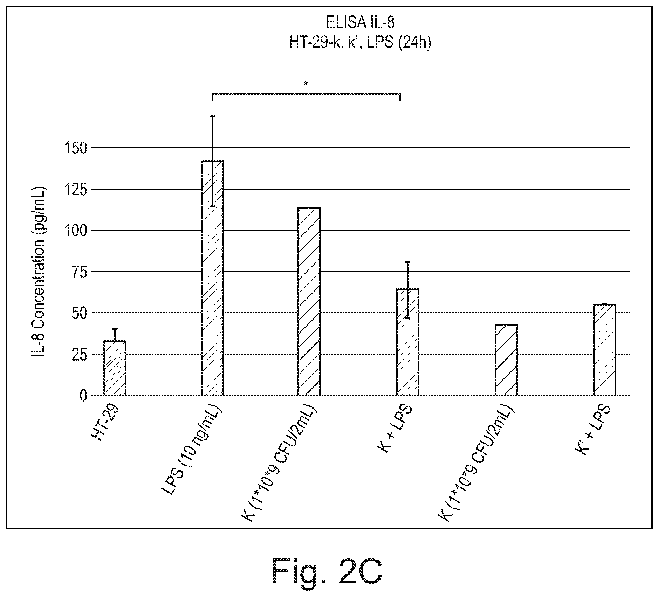

Figure 2 shows the graphs of the anti-inflammatory activity of *Lactobacillus kefiri* in co-culture with the intestinal epithelial cells HT-29 stimulated with LPS. A) Cell viability expressed as number of viable cells, B) cell viability expressed as % of survival, C) IL-8 assays in untreated samples (NT control), in samples stimulated with LPS (10 ng/mL) and in samples treated with viable *Lactobacillus kefiri* and thermally inactivated (10×10⁺⁹ CFU/well).

### DETAILED DESCRIPTION OF THE INVENTION

The object of the present invention is to provide a bacterial strain belonging to the *Lactobacillus kefiri* species, wherein said strain is *L. kefiri* SGL 13 deposited with accession number DSM 27331 on 13/06/2013 at the International Depositary Authority Leibniz-Institut DSMZ - Deutsche Sammlung von Mikroorganismen und Zellkulturen GmbH.

In a further aspect, the invention concerns a composition comprising the bacterial strain *L. kefiri* SGL 13, optionally added with pharmacologically acceptable excipients.

The bacterial strain *L. kefiri* SGL 13 is advantageously prepared in the form of a composition. Suitable compositions may be in the form of powders, capsules, tablets, granulates, drops and syrups, chewing gum. The ranges of use of the ferment are from 1 billion/dose to 20 billion/dose, preferably such a range is from 200 billion/dose to 20 billion/dose, more preferably from 50 billion/dose to 40 billion/dose.

The invention further concerns a composition comprising the bacterial strain *L. kefiri* SGL 13, and a plant extract.

In a preferred embodiment, said plant extract is an extract of *Andrographis paniculata,* Turmeric, *Boswellia serrata,* tocotrienols (extracted also from vegetables rich in vitamin E having antioxidant activity), fennel (*Foeniculum vulgare*), licorice (*Glycyrrhiza glabra*), ginger (*Zingiber officinale*), Lemon Balm (*Melissa officinalis*), Marshmallow (*Althaea officinalis*), mint (*Mentha L*.), thyme (*Thymus L*.) and rosemary (*Rosmarinus officinalis,* essential oils OE or dry extracts ES), green tea.

In an even more preferred embodiment, said plant extract is an extract of *Andrographis paniculata.*

The possible compositions may simple be premixed powders, finished pharmaceutical forms: capsules, tablets, sachets, drops and syrups, chewing gum.

The ranges of use of the ferment are from 1 billion/dose to 20 billion/dose for the ferment, and from 0.1 g/dose to 1 g/dose for the extract of *Andrographis paniculata.* In the present invention, when using the definition:
- "prebiotic" means any substance present in food that is not absorbed by the body, but it is used by the intestinal flora. Most prebiotics are carbohydrates, in particular oligosaccharides. Prebiotics promote growth and activity of bacterial species important for the digestive health of the host organism; and
- "probiotic" means a living, non-pathogenic microorganism present in food or added to it which, when administered in adequate amounts, brings a benefit to the health of the host. Lactic acid bacteria (LAB, Lactic Acid Bacteria), mostly represented by lactobacilli and bifidobacteria, are the most common types of probiotic microorganisms.

Advantageously, the composition comprising the bacterial strain *L. kefiri* SGL 13 may further comprise one or more prebiotics or said composition may further comprise a plant extract and one or more prebiotics.

In a preferred embodiment, said prebiotic is selected from the group consisting in GOS (gluco-oligosaccharides), FOS (fructo-oligosaccharides), Guar Fiber, Inulin, MOS (mannan-oligosaccharides), pectins, Glucomannan.

Advantageously, the compositions comprising:
- the bacterial strain *L. kefiri* SGL 13;
- the bacterial strain *L. kefiri* SGL 13 and a plant extract of *Andrographis paniculata;*
- the bacterial strain *L. kefiri* SGL 13 and one or more prebiotics;
- the bacterial strain *L. kefiri* SGL 13, and a plant extract of *Andrographis paniculata* and one or more prebiotics, have probiotic activity.

The compositions according to the invention may be in a solid or liquid form. In the compositions according to the present invention, the dosages are in a range from 1 billion/dose to 20 billion/dose for ferments, preferably this range is from 200 billion/dose to 20 billion/dose, more preferably from 50 billion/dose to 40 billion/dose, in a range from 0.1 g/dose to 5 g/dose for the prebiotics, and in a range from 0.1 g/dose to 1 g/dose for the extract of *Andrographis paniculata.*

The compositions according to the invention can be employed for use as a medicament.

The compositions according to the present invention have an anti-inflammatory, immunomodulatory and antitumor action, in fact they allow to modulate the immune and inflammatory responses at the level of the intestinal mucosa and at a systemic level, in order to guarantee the maintenance of the gastrointestinal homeostasis thus preventing the onset of intestinal cancer diseases.

Suitable compositions may be in the form of powders, capsules, tablets, granulates, drops and syrups, chewing gum.

In particular, said medicament is for use in the treatment and prevention of intestinal inflammation and in the treatment and prevention of intestinal neoplasms. In particular, said intestinal inflammations may be selected from the group consisting of ischaemic colitis, lymphocytic colitis, ulcerative colitis, diversion colitis, paucities, Chron's disease, and enterocolitis such as necrotizing enterocolitis and all acute and chronic intestinal inflammatory diseases in general (IBDs).

Therefore, the present work relates to the identification and characterization of the probiotic properties of the *L. kefiri* SGL 13 strain and use thereof for the therapeutic purposes specified above.

The compositions according to the invention can be further employed for food use and as a supplement.

Suitable food compositions may be in the form of a beverage, preferably fruit juice, coffee, tea, and a soft drink.

The invention further concerns a medical or pharmaceutical device comprising the compositions comprising:
- the bacterial strain *L. kefiri* SGL 13;
- the bacterial strain *L. kefiri* SGL 13 and a plant extract of *Andrographis paniculata;*
- the bacterial strain *L. kefiri* SGL 13 and one or more prebiotics; and
- the bacterial strain *L. kefiri* SGL 13, a plant extract of *Andrographis paniculata* and one or more prebiotics.

The compositions comprising the associations of *L. kefiri* SGL 13 and plant extract of *Andrographis paniculata* and one or more prebiotics allow to enhance the beneficial effects of the formulation resulting from the association.

Examples of embodiments of the present invention are given below by way of illustration.

### EXAMPLES

### Example 1: Strain Isolation

The strain was isolated from kefir granules. The granules were resuspended in milk, incubated for 30 minutes at 37°C on selective LAMVAB (LV) medium for vancomycin-resistant lactobacilli.

To obtain 1 liter of LAMVAB medium, the solution A was prepared by dissolving the following components in 500 mL of distilled water:

| | |
|---|---|
| MRS Broth | 52.2 g/L |
| L-Cysteine | 0.25 g/L |
| Bromocresol Green (0.25% in ethanol) | 3 ml/L |

Solution A was brought to pH 5 with 4 N HCl and sterilized in autoclave at 110°C for 30 minutes. Subsequently, the solution B was prepared by re-suspending 18 g of agar in 500 mL of distilled water, also sterilized at 110°C for 30 minutes. After being cooled down to 55°C, solutions A and B were joined. Before being poured into the plate, agar medium was added with 20 mg/L vancomycin.

### RESULTS:

The strain was isolated from kefir granules by using selective LAMVAB (LV) medium for vancomycin-resistant lactobacilli. After incubation at 37°C in anaerobiosis, o/n, different types of colonies were observed on the LV plates; those with a rod-shaped morphology under an optical microscope were rubbed again on MRS agar plates added with 0.05% cysteine, in order to obtain isolated cultures each of a single strain from each other.

*L. kefiri* colonies are medium size, opaque with a whiter central dot in the center, regular and convex colonies; the morphology of the microorganism under the microscope can be described as micro-organisms in the shape of a short, thick and regular rod.

### Example 2: Molecular characterization

The isolated strain was initially identified by growing it on a selective LAMVAB medium, as described in the previous paragraph, and direct observation of the morphology of the plate-grown colonies and cell morphology following observation by light microscopy.

The certain attribution of the genus and the species was carried out, instead, by techniques of analysis of 16S ribosomal DNA and biochemical characterization.

### Results:

Lactobacilli strains selected by isolation with the LV medium were taxonomically classified by the study of the entire 16S gene. A region about 1500 bp long was amplified using generic primers for eubacteria, sequenced, and then aligned using BLAST with public databases. The results enabled a certain classification with its ascription to the *Lactobacillus kefiri* species. The Maldi Tof results shown below provided additional support to the strain identification.

| Analyte Name | Analyte ID | Organism (best match) | Score Value | Organism (second best match) | Score Value |
|---|---|---|---|---|---|
| D2 (+++)(A) | 7 | Lactobacillus kefiri | 2.34 | Lactobacillus kefiri | 2.28 |
| D3 (++)(A) | 22 | Lactobacillus kefiri | 2.035 | Lactobacillus kefiri | 1.94 |
| D4 (++)(A) | 23 | Lactobacillus kefiri | 2.257 | Lactobacillus kefiri | 2.104 |

The *L. kefiri* SGL 13 strain was deposited at the European International Collection DSMZ, an organization recognized with the IDA (*International Depositary Authority*) status, with a "patent deposit" having the following data: *L. kefiri* SGL 13 DSM 27331, on 13/06/2013.

In addition, the microorganism was amplified with species-specific primers, verifying the positivity by the appearance of the amplicon of the expected size of 500 bp.

This additional data allows us to unambiguously attribute the species, and to have a control and monitoring tool for the culture under examination during the fermentation and lyophilization phases. Figure 1 shows the result of amplification of *L. kefiri* SGL 13 with species-specific primers.

### Analysis of sequence 16S

The microorganism genome was extracted using commercial kits (Macherey Nagel, Düren, Germany) following manufacturer's indications. DNA was used in the concentration of 50-100 ng for the amplification reaction of 16S gene. Amplification reactions were performed using Biometra T-professional thermocycler (Biometra, Göttingen, Germany). The amplification conditions used were those that gave the best yields of amplified gene. All reactions were carried out in a final volume of 25 µL.

The components used in all amplification reactions are:
- DyNAzyme (Finnzymes, Espoo, Finland) as thermostable DNA polymerase (1 U per reaction) and its reaction buffer 10× Optimized DyNAzyme buffer (100 mM Tris-HCl, 500 mM KCl, 15 mM MgCl₂, 0.1% Triton X-100, pH 8.8 at 25°C);
- mixture of triphosphate nucleotides (8 mM, Euroclone, Siziano, Italy).
- primer set(100 µM, Eurofins MWG Operon, Ebersberg, Germany);
- genomic DNA;
- sterile water.

The following are the primers used, the thermocycle and the expected amplicon.
Primer: 16S-27 For 5'-AGAGTTTGATCCTGGCTCAG-3' (SEQ ID NO:1)
16-1552 Rev 5'-AAGGAGGTGWTCARCCGCA-3' (SEQ ID NO:2)

| | | |
|---|---|---|
| Thermocycle: | 95°C 5' | 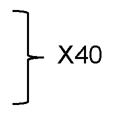 |
| | 95°C 40" | |
| | 55°C 40' | |
| | 72°C 1' | |
| | 72°C 5' | |
| | 15° C ∞ | |
| Amplicon: | 1552 bp | |

The fragment of about 1550 bp was sequenced (MWG Biotech, Ebersberg, Germany) and was aligned with Blast database (NCBI, nucleotide blast) to obtain the correct identification of the species.

### Species-specific PCR

The same DNA was used as a template for a species-specific primer set for *L. kefiri* SGL 13. These primers produce a fragment of about 500 bp if the amplified DNA belongs to the species in question.

The primers, the thermocycle and the amplicon are shown below.
Primer: Lk1 5'-CAACAATCAAAGGGGTTGTTG-3' (SEQ ID NO:3)
Lk2 5'-TCACTAGGAGTAATTGAACCA-3' (SEQ ID NO:4)

| | |
|---|---|
| Thermocycle: | 95°C 10' |
| 95°C 1' | 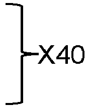 |
| 48°C 1' | |
| 72°C 1' | |
| 72°C 5' | |
| 15°C ∞ | |
| Amplicon: | 500 bp |

The visualization of the amplification products was carried out by electrophoretic run on agarose gel (1-2%). This analysis was carried out by loading on the gel a volume of the reaction mixture of 10-15 µL, in parallel with the molecular weight marker (MassRuler^{™} Low Range DNA Ladder, Fermentas).

### Strain profile in MALDI-TOF MS

To obtain an identifier Maldi Tof profile, the sample was prepared as follows:
a bacterial culture of *L. kefiri* SGL 13 grown overnight was collected in order to have a sufficient number of cells for detection (5 billion). The cell pellet was washed with saline solution to remove residues of fermented medium and resuspended in 300 µL of distilled water and 900 µL of pure ethanol. The suspension was well homogenized. The sample was then centrifuged at 13,000 rpm for 2 minutes and air-dried after removing the supernatant. The sample was stored at -20°C pending analysis.

At the time of analysis, the sample was resuspended with 50 µL of 70% formic acid. After resuspension, additional 50 µL of acetonitrile were added and the whole was vigorously stirred. The sample was then centrifuged for 2 minutes at 13,000 rpm. 1 µL of supernatant was placed onto the center of a spot of a disposable Maldi plate and air-dried. The addition of 1 µL of matrix made of organic materials (α-cyano-4-hydroxycinnamic acid) started the Maldi Tof analysis.

Maldi Tof MS was performed with a MicroFlex LT system using the conditions included in the user manual. The ions generated by a nitrogen laser, at a 337 nm wavelength, were linearly captured in a mass range of 2 to 20 KDa. The instrument is connected to the Biotyper 2.0 software (Bruker Daltonics) capable of acquiring incoming data, elaborate them in mass spectra and compare them with reference spectra present in the internal database (3,740 spectra of 319 genres and 1,946 different species). For each 24-sample plate, a standard test was included to calibrate and validate the analysis.

The degree of correspondence with the typical spectrum for each microorganism present in the database determines the attribution of a so-called "score value" expressing the degree of certainty for the proposed identification of the species under examination. Scores with a value greater than 2,000 are considered reliable for identification at the species level, values between 1,700 and 1,900 give a certain identification at the genus level.

The strain was analyzed in triple on 3 different bacterial cultures.

### Genome Sequencing

*L. kefiri* SGL 13 genome was fully sequenced by preparing two libraries analyzed on the Illumina HiSEq2500 platform and on the PacBio RS platform.

The strain was grown in MRS medium at 37°C per 24 ore in order to extract the DNA. Genomic DNA was extracted with the Wizard purification kit (Promega), according to the manufacturer's instructions.

Sequencing was conducted on the HiSeq 2500 platform (Illumina) with Nextera XT protocol. The sequences in FASTQ format obtained by sequencing a paired-end library on the HiSeq2500 platform were filtered through Casava 1.8.3 by Illumina. The quality analysis was performed on data previously filtered through Illumina Chastity. Subsequently, reads containing the PhiX control were eliminated through an internal protocol. Furthermore, reads containing the (partial) sequence of the adapter were trimmed, maintaining a minimum readable reads length of 50 bp. The second quality control was performed using FASTQC software (version 0.10.0). The data obtained from genome sequencing using PacBio RS instrumentation were processed and filtered using SMRT Analysis software suite. Continuous Long Read (CLR) data were filtered based on length (>35) and Reads quality (>0.75). Final data regarding the statistical analysis of the Reads produced thanks to PacBio.

The first step to obtain a de-novo genome assembly is the improvement of the quality of the Illumina FASTQ sequences by trimming low quality bases thanks to the program bbduk, a component of the BBMap 34.46 programs suite. The sequences thus filtered were combined and aggregated in contigs using ABySS 1.5.1. The contigs were linked up and placed inside super-scaffolds thanks to CLR reads obtained from PacBio, which provide information on the continuity of each contig with respect to the others, within the genome. This alignment operation was performed with BLASR. The estimate of orientation, order and distance between individual contigs was obtained using SSPACE-LongRead scaffolder 1.0. The missing regions within the super-scaffolds were (partially) closed automatically using GapFiller 1.10 program. The method exploits the size of the insert between the reads obtained by sequencing on Illumina the platform.

The genome was annotated using the Prokka software.

### Results:

*L. kefiri* SGL 13 genome was assembled in 10 final scaffolds, it has an approximate size of about 2,408,480 bp with an average GC content of 41.9%. The genome contains 3271 genes of which 1609 have a known function. Furthermore, 37 families of active carbohydrate enzymes (CAZymes) genes and a single bacitracin resistance gene, that is not found on a transposable element, were identified. As a result, there is no risk of transferability of antibiotic resistance to pathogenic microorganisms.

The European Food Safety Authority (EFSA) document on probiotic safety recommends that commercial strains do not exhibit transferable antibiotic resistance. Therefore SGL 13 can be considered safe for human consumption and may be used in combination with antibiotics without any risk of transferability of antibiotic resistance and, due to its probiotic properties, this strain represents a good candidate for the production of a product based on this lactic ferment.

### Example 3: Biochemical and phenotypic characterization

### Gram Staining

Gram staining is a type of differential staining that allows to classify bacteria in Gram-positive and Gram-negative.

This identification test, which was carried out using the Gram Color Kit (Liofilchem, Teramo, Italy), is divided into 4 steps, spaced by washings with distilled water, each of them involving treatment with a particular reagent that is allowed to act for about 1 minute on a slide surface on which a loop of cells from a culture on solid medium, dissolved with a drop of sterile water, was previously deposited. The suspension thus obtained was heat fixed with a Bunsen burner.

The reagents used, in order of application, are:
- Crystal violet,
- Lugol's solution,
- Decolorizing solution (ethyl alcohol),
- Safranin.

At the end, Gram-positive bacteria appear purple in color, while Gram-negative bacteria are red or pink.

### Results:

The biochemical profile of the *L. kefiri* SGL 13 strain was also analyzed by Gram staining, catalase test, oxidase test and API 50CH system.

The results obtained confirmed that the strain is gram positive, catalase and oxidase negative, and ferment the sugars as shown in Table 1.

**Table 1: Biochemical characterization of L. kefiri SGL by 13 by API 50CH.**

| | | | | |
|---|---|---|---|---|
| Arbutin | NEG | | Potassium 5-ketogluconate | NEG |
| Amygdalin | NEG | | Potassium 2-ketogluconate | NEG |
| N-acetylglucosamine | NEG | | Potassium gluconate | POS |
| Methyl-αD-glucopyranose | NEG | | L-arabitol | NEG |
| Methyl-αD-mannopyranose | NEG | | D-arabitol | NEG |
| D-sorbitol | NEG | | L-fucose | NEG |
| D-mannitol | NEG | | D-fucose | NEG |
| Inositol | NEG | | D-tagatose | NEG |
| Dulcitol | NEG | | D-lyxose | NEG |
| L-rhamnose | NEG | | D-turanose | NEG |
| L-sorbose | NEG | | Gentiobiose | NEG |
| D-mannose | NEG | | Xylitol | NEG |
| D-fructose | NEG | | Glycogen | NEG |
| D-glucose | NEG | | Starch | NEG |
| D-galactose | NEG | | D-raffinose | NEG |
| Methyl-βD-xylopyranose | NEG | | D-melezitose | NEG |
| Adonitol | NEG | | Inulin | NEG |
| L-xylose | NEG | | D-trehalose | NEG |
| D-xylose | NEG | | D-sucrose | NEG |
| D-ribose | PCS | | D-melibiose | NEG |
| L-arabinose | POS | | D-lactose | NEG |
| D-arabinose | NEG | | D-maltose | NEG |
| Erythritol | NEG | | D-cellobiose | NEG |
| Glycerol | NEG | | Salicin | NEG |
| Control | NEG | | Esculin | NEG |

### Catalase Assay

This assay serves to verify the presence of catalase enzyme, able to detoxify hydrogen peroxide, in a bacterial culture.

A loop of cells from a culture on solid medium were dissolved in a drop of 30% hydrogen peroxide. Immediate appearance of effervescence is indicative of catalase presence.

Effervescence is due to the oxygen that develops in the reaction:

2 H²O² →H²O + O²

### Oxidase Assay

The oxidase assay is an enzymatic test used to detect the presence of cytochrome oxidase enzyme in bacteria. It is positive for aerobic and facultative anaerobic bacteria. Oxidase Test Discs (Liofilchem, Teramo, Italy), i.e. 3 mm diameter paper discs impregnated with tetramethyl-p-phenylenediamine dihydrochloride (Kovacs reagent), were used. The disc was moistened with 2 drops of distilled water and rubbed with a loop of a pure colony.

The development of a blue color within 30 seconds demonstrates the oxidation of the substrate contained in the disk. In this case, the microorganism is oxidasepositive, conversely, if no color development occurs, the microorganism will be oxidase-negative.

### Sugar Fermentation Profile (API)

For the biochemical characterization of *L. kefiri* SGL 13, the API 50 CH gallery system was used with API 50 CHL (BioMérieux, Milano, Italy) inoculum medium. The gallery consists of 50 microtubes containing dehydrated substrates for detection of enzymatic activity and fermentation of sugars. The bacterial suspension, having a turbidity of 2 McFarland, was distributed in the microtubes, and anaerobiosis was obtained by covering them with sterile paraffin oil. Substrate metabolism by the bacterium during the incubation period at 37°C is highlighted by a color change in the medium, following acidification of the medium. Reading of the reaction results was performed on the basis of a reading table where the results were recorded, and the identification was performed using a computer software (www.apiweb.biomerieux.com).

### Example 4: Probiotic characteristics of L. kefiri SGL 13

### Tolerance to gastro-intestinal environment

In order to evaluate the tolerance to gastro-intestinal juices, viability of the isolated strain over time was determined by plate count, after it had been contacted with:
- Synthetic pepsin;
- Synthetic pancreatin;
- Various concentrations of bile salts;
- Various concentrations of NaCl;
- Acid pH growth medium.

### - Pepsin Resistance Test

A pepsin solution consisting of 3 g/L of pepsin, derived from pig gastric mucosa (Sigma-Aldrich, St. Louis, MO), was obtained by dissolving powdered pepsin in 0.5% NaCl (w/v) sterile saline solution, and the pH was adjusted to 3. Starting from cultures grown overnight in 10 mL of MRS broth medium supplemented with 0.05% of L-cysteine, at 37°C for about 18 hours, in the presence of O₂, about 10⁹ cells/mL were harvested by centrifugation at 4,000 rpm for 10 minutes. Subsequently, the cells thus pelleted were washed with a 0.09% NaCl (w/v) sterile saline solution and subjected to the same centrifugation cycle. The washing was repeated a second time under the same conditions.

To simulate passage through the gastric tract, cells were resuspended in 10 mL of pepsin solution at pH 3. The suspension thus prepared was incubated in anaerobiosis at 37°C for 2 hours, measuring the number of CFU/mL at 0, 90 and 120 minutes by plate count.

To monitor growth in the absence of pepsin, the strain under examination was inoculated into two 0.5% (w/v) sterile saline solutions (w/v), at pH 3, one containing pepsin and one without it (control sample). The samples were incubated under the same conditions described above.

The resistance of the treated strain was expressed as a percentage of survival over the control.

### - Pancreatin Resistance Test

A pancreatin solution, consisting of 1 g/L pancreatin from pig pancreas (Sigma-Aldrich), was obtained by dissolving the powder in 0.5% NaCl (w/v) sterile saline solution, and the pH was adjusted to 8. Starting from cultures grown overnight in 10 mL of MRS broth medium supplemented with 0.05% of L-cysteine, at 37°C for about 18 hours in the presence of O₂, about 10⁹ cells/mL were harvested by centrifugation at 4,000 rpm for 10 minutes. With the same centrifugation cycle, the cells were washed with a 0.09% NaCl (w/v) sterile saline solution.

Subsequently, the cells were neutralized by washing with a sterile phosphate buffer (PBS - 8 g/L NaCl, 0.2 g/L KCl, 1.44 g/L Na₂HPO₄, 0.24 g/L KH₂PO₄) at pH 7 and, to simulate passage through the intestine, they were resuspended in 10 mL of pancreatin solution at pH 8. The suspension was incubated in anaerobiosis at 37°C for 4 hours, and the number of CFU/mL at 0, 60 and 240 minutes was estimated by plate count.

To monitor growth in the absence of pancreatin, the strain under evaluation was inoculated into two 0.5% (w/v) sterile saline solutions, at pH 8, one containing pancreatin and one without it (control sample). The samples were incubated under the same conditions described above.

The resistance of the treated strain was expressed as a percentage of survival over the control.

### - Resistance test to bile salts, pH and NaCl

The analysis was carried out on bacterial cultures grown overnight. Approximately 10⁹ cells/mL were harvested by centrifugation at 4,000 rpm for 10 minutes. The cells were then washed with 2 mL of sterile physiological solution (NaCl 9 g/L) and, following centrifugation at 4,000 rpm for 10 minutes, they were resuspended in MRS broth containing:
- 0%, 0.5%, 1%, 2%, 3% bile salts (w/v, Sigma-Aldrich);
- 0%, 2%, 6%, 10% di NaCl (w/v, Oxoid);
- pH 6.3, 3.5, 2, 1.5 adjusted with 4 N HCl.

The solutions were incubated in anaerobiosis at 37°C for 24h; for each inoculum the optical density at 600 nm was recorded at 0h, 2h and 24h, while the estimate of the CFU number/mL was performed at 0h and 24h, by plate count.

### Results: probiotic characteristics of L. kefir SGL13

### Tolerance to gastro-intestinal environment

The microorganism was tested in order to ensure its gastro-intestinal transit and guarantee it could survive and reach, remaining fully viable, the intestinal section where it must perform its physiological activity.

This strain was therefore tested under the following environmental conditions:
1. Resistance to pepsin in acid environment
2. Resistance to pancreatin in basic environment
3. Resistance to high bile salts concentration
4. Resistance to acid environment
5. Resistance to high salts concentrations

*L. kefiri* SGL 13 showed a good tolerance to the gastro-intestinal environment. In particular, the results showed a good survival rate after 1h, and also after 4h of incubation, in the presence of pancreatin at pH 8. Similar results were obtained after 1 hour and 30 minutes and after 2 hours and 30 minutes of incubation, in the presence of pepsin at pH 3. The results obtained are shown in Table 2 below:

**Table 2: L. kefiri SGL 13 resistance to pepsin and pancreatin**

| | **LOG₁₀ CFU/mL *L. kefiri* SGL 13** | | |
|---|---|---|---|
| **PEPSIN pH 3** | **Test** | **t0** | 8.13±0.01 |
| | | **t90'** | 8.09±0.04 |
| | | **t120'** | 8.14±0.05 |
| | | **% Survival @90'** | 99.53±7 |
| | | **% Survival @120'** | 100.1315 |
| | **Control** | **t0** | 7.96±0.02 |
| | | **t90'** | 7.31±0.03 |
| | | **t120'** | 6.98±0.01 |
| | | **% Survival @90'** | 91.78±8 |
| | | **% Survival @120'** | 87.6216 |
| **PANCREATIN pH 8** | **Test** | **t0** | 8.48±0.25 |
| | | **t60'** | 8.08±0.27 |
| | | **t240'** | 7.77±0.27 |
| | | **% Survival @60'** | 95.3517 |
| | | **% Survival @240'** | 91.5916 |
| | **Control** | **t0** | 8.04±0.28 |
| | | **t60'** | 7.71±0.25 |
| | | **t240'** | 7.82±0.27 |
| | | **% Survival @60'** | 95.8718 |
| | | **% Survival @240'** | 97.22516 |

The microorganism is slightly affected by the presence of bile salts at concentrations higher than 2% (about 10 times higher than physiological ones), showing nevertheless a good survival even in the presence of 3% of the same. Results are shown in Table 3. This makes it suitable for use as a probiotic.

**Table 3: L. kefiri SGL 13 resistance to bile salts**

| **LOG₁₀ CFU/mL *L. kefiri* SGL 13** | | | |
|---|---|---|---|
| **Bile Salts** | **t0** | **t24h** | **Growth** |
| **0%** | 6.65±0.28 | 7.90±0.27 | Normal |
| **0.5%** | 6.82±0.26 | 7.25±0.25 | Normal |
| **1%** | 6.69±0.28 | 7.23±0.28 | Normal |
| **2%** | 6.46±0.27 | 6.89±0.28 | Slight decrease |
| **3%** | 6.48±0.27 | 6.85±0.25 | Slight decrease |

### Resistance to acid environment

Survival of *L. kefiri* SGL 13 in acid environment is compromised within 24 hours at a pH below 3, while at pH 3 there is a reduction in growth of about 1 logarithmic unit, as shown in Table 4.

**Table 4: L. kefiri SGL 13 resistance to acid environment**

| **LOG₁₀ CFU/mL *L. kefiri* SGL 13** | | | |
|---|---|---|---|
| **pH** | **t0** | **t24h** | **Growth** |
| **1.5** | <5 | <2 | No growth |
| **2** | <5 | <2 | No growth |
| **3** | 7.62±0.10 | 6.12±0.25 | 1 Log decrease |
| **6.3** | 7.39±0.02 | 7.99±0.03 | Normal |

### Resistance to high salts concentrations

As shown in Table 5, *L. kefiri* SGL 13 shows a good tolerance to high salt concentrations, as its survival is guaranteed up to 6% of NaCl, at higher concentrations (10%) the growth rate undergoes a decrease of about one logarithmic unit.

**Table 5: L. kefiri SGL 13 resistance to high salts concentrations**

| **LOG₁₀ CFU/mL *L. kefiri* SGL 13** | | | |
|---|---|---|---|
| **NaCl** | **t0** | **t24h** | **Growth** |
| **0%** | 6.31±0.10 | 8.18±0.15 | Normal |
| **2%** | 6.48±0.06 | 7.95±0.10 | Normal |
| **6%** | 6.48±0.05 | 6.26±0.18 | Slight decrease |
| **10%** | 6.55±0.06 | 5.37±0.20 | 1 Log decrease |

### Example 5: Adhesion to Intestinal Cells Monolayer

To assess the adhesion activity of the *L. kefiri* SGL 13 strain to the human enterocyte line HT29, 1 × 10⁸ bacterial cells were added to an epithelial monolayer of HT29 cells (1.23 × 10⁶ cells) and incubated for 1.5 h. At the end of the incubation, the cell monolayer was washed 4 times with PBS, and the adherent bacterial cells were quantified by Real Time PCR using genus-specific primers (Candela M, Seibold G, Vitali B, Lachenmaier S, Eikmanns BJ, Brigidi P. Real-time PCR quantification of bacterial adhesion to Caco-2 cells: competition between bifidobacteria and enteropathogens. Res. Microbiol. (2005) 156:887-895*).*

The adherent enteropathogens *Salmonella Typhimurium* and *E. coli* ETEC H10407 were used as positive controls, while the non-adherent *E. coli* B44 strain was used as negative control.

### Results: Adhesion to Intestinal Cells Monolayer

The adhesion activity of the *L. kefiri* SGL 13 strain to the human enterocyte line HT29 was evaluated by counting the number of microorganisms adhered to HT29 cells by Real time PCR. The result obtained was 9.32E+3/100 HT29 cells, *L. kefiri* SGL 13 may be therefore considered a strongly-adhesive strain (adhesion reference value *Salmonella* 6.77E+5/100 HT29 cells and 157 of *E. coli* B44/100 HT29 cells).

### Example 6: Antibiotic Resistance Profile

Sensitivity to antimicrobial agents of the *L. salivarius* SGL03 strain was tested using MIC Test Strip (Liofilchem), a quantitative method for determination of the minimum inhibitory concentration (MIC) of a single antimicrobial agent against microorganisms. The kit consists of paper strips impregnated with a predetermined gradient of antibiotic concentrations, consisting of 15 dilutions in the range of dilutions used in conventional methods for MIC determination.

The cultures in stationary phase were diluted in MRD buffer (Liofilchem) in order to have a turbidity equal to McFarland 3 standard. 100 µL of this solution were spatulated on the surface of MRS agar medium supplemented with 0.05% of L-cysteine in plate, in order to produce a homogeneous growth. After allowing the agar surface to dry completely, the strip was placed in the center of the plate with the MIC values facing upward. The plates thus prepared were incubated, with the lid facing upward, in aerobiosis, at 37°C for 48 hours.

When the kit strip is applied to the inoculated surface of plated agarized medium, the pre-defined gradient is released by the strip to the medium, therefore, after the incubation, an elliptical, symmetrical, and centered along the strip inhibition area can be observed. The MIC value, expressed in µg/mL, was read at the intersection point between the edge of the inhibition ellipse and the paper strip.

### Results: Antibiotic Resistance Profile

Table 6 reported below shows the resistance profile to antibiotics tested against the L. *kefiri* SGL 13 strain, by using the MIC Test Strip.

**Table 6: L. kefiri SGL 13 antibiotic resistance profile**

| **ANTIBIOTIC** | **FAMILY** | **MECHANISM OF ACTION** | **MIC (µg/mL)** |
|---|---|---|---|
| Amoxicillin | β-lactamase | Wall synthesis inhibitor | 0.38 |
| Ampicillin | β-lactamase | Wall synthesis inhibitor | 0.19 |
| Clindamycin | Lincosamide | Protein synthesis inhibitor | 0.023 |
| Chloramphenicol | Macrolide | Protein synthesis inhibitor | 3 |
| Ciprofloxacina | Quinolone | DNA synthesis inhibitor | >32 |
| Erythromycin | Macrolide | Protein synthesis inhibitor | 0.19 |
| Gentamycin | Aminoglycoside | Protein synthesis inhibitor | 1 |
| Kanamycin | Aminoglycoside | Protein synthesis inhibitor | 32 |
| Rifampicin | Rifampicin | Protein synthesis inhibitor | 0.19 |
| Streptomycin | Aminoglycoside | Protein synthesis inhibitor | 16 |
| Sulfamethoxazole | Sulfamide | DNA synthesis inhibitor | 12 |
| Tetracycline | Tetracycline | Protein synthesis inhibitor | 96 |
| Vancomycin | Glycopeptide | Wall synthesis inhibitor | >256 |
| Claritromicina | Macrolide | Protein synthesis inhibitor | 0.047 |
| Minociclina | Tetracycline | Protein synthesis inhibitor | 1.5 |
| Doxiciclina | Tetracycline | Protein synthesis inhibitor | 16 |
| Ceftriaxone | β-lactamase | Wall synthesis inhibitor | 0.5 |
| Cefixime | β-lactamase | Wall synthesis inhibitor | 0.5 |
| Cefuroxima | β-lactamase | Wall synthesis inhibitor | 64 |

### Example 7: Evaluation of the anti-inflammatory activity of L. kefiri SGL 13: II-8 assays

The anti-inflammatory capacity of *L. kefiri* SGL 13 was tested using the human enterocyte line HT-29. First, the appropriate dose of LPS in order to obtain an inflammatory response with this cell line was evaluated. So, a dose-response curve was performed by growing HT-29 cells in DMEM, with 10-15% fetal bovine serum, 1% L-glutamine, 1% Penicillin and Streptomycin, at 37°C and 5% CO2 for 2-3 days. At confluence, the cells were stimulated with increasing LPS concentrations from 1 ng/mL to 1000 ng/mL for 22 hours

After the concentration of 10 ng/mL was identified as optimal, co-culture tests were carried out, in which the confluent HT-29 cells were preincubated for 2 hours with *Lactobacillus kefiri* 1×10⁺⁹ CFU/well, both as viable and thermally inactivated form, and subsequently stimulated with LPS for another 22 hours.

Determination of the IL-8 released was quantified by ELISA (Sigma Aldrich).

Results: Evaluation of the anti-inflammatory activity of *L. kefiri* SGL 13: II-8 assays The anti-inflammatory activity of *L. kefiri* SGL 13 was tested using the human enterocyte line HT-29 by evaluating the release of IL-8. As shown in Fig. 2, the untreated cells (NT) have a basal production of IL-8 equal to 30 pg/mL, after stimulation with LPS for 22 hours there is an increase of IL-8 greater than 4 times. Pretreatment with *L. kefiri,* both in viable and non-viable form, significantly reduces production of IL-8 by about half. This demonstrates how the treatment with *L. kefiri* SGL 13 results in a more moderate inflammatory response as compared to when it is not present.

### Example 8: Animal experimentation: association of L. kefiri SGL 13 and extract of Andrographis paniculata in the prevention and treatment of intestinal inflammations

Experimentation aims to evaluate di efficacy of a supplement prototype based on *L. kefiri* SGL 13 and a plant extract of *Andrographis paniculata* in reducing intestinal inflammation induced in C57BL/6 mice by administration of 1.5% dextran sodium sulfate (DSS) in drinking water.

Considering the beneficial properties documented in the literature of the probiotic *L. kefiri* and the plant extract of *Andrographis paniculata,* the present project aims to evaluate the efficacy of their association as a useful supplement and aid to traditional therapies for intestinal inflammations.

Their synergy could increase the effectiveness of their individual actions, filling the limits of those treatments involving only the use of one or the other.

For this purpose, 40 mice divided into 4 experimental groups will be used: first group (negative control), second group (toxicity test) fed with supplement prototype, third group (positive control) subjected to treatment with 1.5% DSS and fourth group (effectiveness test) subjected to treatment with 1.5% DSS plus supplement prototype.

The experimentation will last 38 days in total and will be organized into 4 separate phases: acclimation of the animals to housing conditions (1 week), administration of the supplement prototype (1 week), inflammation induction using 1.5% DSS (9 days), interruption of the treatment with 1.5% DSS (2 weeks) and animals sacrifice. The objects that the project aims to achieve are as follows:
1. Verify the absence of adverse effects and the tolerance of the supplement prototype based on the lactic ferment *Lactobacillus kefiri* SGL 13 and plant extract of *Andrographis paniculata.*
2. Evaluate the induction of intestinal inflammation obtained by the administration of 1.5% dextran sodium sulfate (DSS) and describe the various phases (acute, chronic, remission) and duration thereof.
3. Characterize the intestinal microbiota and verify the colonization by the administered lactic ferment *lactobacillus kefiri* SGL 13.
4. Evaluate the production (amount and type) of short chain fatty acids (SCFAs) by the intestinal microflora.
5. Evaluate the efficacy of the supplement prototype in reducing the levels of inflammation induced by DSS.
6. Post-mortem evaluation of the degree of inflammation at the intestinal level by histological analysis.
7. Post-mortem evaluation of any signs of toxicity and anatomo-pathological alterations in the liver and kidneys.

In each experimental phase, feces samples from each animal belonging to the 4 groups will be collected, and a blood sample will be taken as well. Food/water consumption and body weight will also be monitored for all animals.

For the groups subjected to treatment with DSS (Groups 3 and 4) only, the DAI score, and index of progression of the degree of intestinal inflammation based on the simultaneous evaluation of three different parameters: feces consistency, presence of blood and loss of weight, will also be calculated.

At the sacrifice, the following organs will be collected from each mouse: liver, spleen, small intestine, large intestine.

At the hepatic level, the absence of toxicity and alterations following the administration of the supplement prototype will be verified. As regards the spleen, the increase in weight vs. the controls will be assessed. At the level of the small intestine, anatomo-pathological alterations will be evaluated and the secretory IgA assay will be performed, while in the large intestine the following assays will be performed in addition to anatomo-pathological alterations: Myeloperoxidase (MPO), Cyclooxygenase-2 (COX-2), Inducible Nitric Oxide Synthase (i-NOS).

All parameters to be monitored and analyzes to be performed during experimentation are summarized in the Table below.

| **Parameters (feces)** | **Parameters (blood)** | **Anthropometric parameters** | **Organs to be removed** |
|---|---|---|---|
| Intestinal Microbiota | Glycemia | Body weight | Small intestine |
| SCFAs | Haemochrom | | Large intestine |
| Colonization by *L. kefiri* | Lipid profile | | Liver |
| DAI score | Glucidic profile | | Spleen |
| | Cytokine | | |

From the detailed description and the Examples reported above, the advantages achieved by the bacterial strain of the present invention are apparent. In particular, the bacterial strain *Lactobacillus kefiri SGL* 13 has been shown to be surprisingly and advantageously suitable for the preparation of compositions for medical use in the treatment of intestinal inflammations and as compositions with probiotic activity.

## Claims

1. A bacterial strain belonging to the *Lactobacillus kefiri* species, wherein said strain is *L. kefiri* SGL 13 deposited with accession number DSM 27331 on June 13, 2013.

2. A composition comprising the bacterial strain according to claim 1.

3. The composition according to claim 2, further comprising a plant extract.

4. The composition according to claim 3, wherein said plant extract is an extract of *Andrographis paniculata.*

5. The composition according to any one of claims 2 to 4, further comprising a prebiotic.

6. The composition according to any one of claims 2 to 5, and pharmacologically acceptable excipients.

7. The composition according to any one of claims 2 to 6, wherein said composition has probiotic activity.

8. The composition according to any one of claims 2 to 7, wherein said composition is in a solid or liquid form.

9. The composition according to any one of claims 2 to 8, wherein said composition is a food composition.

10. The composition according to any one of claims 2 to 9, for use as a medicament.

11. The composition according to any one of claims 2 to 10, for use in the prevention and treatment of intestinal inflammations, and in the treatment and prevention of intestinal neoplasms.

12. The composition for use according to claim 11, wherein said intestinal inflammations are selected from the group consisting of ischaemic colitis, lymphocytic colitis, ulcerative colitis, diversion colitis, paucities, Chron's disease, and enterocolitis, such as necrotizing enterocolitis, and all acute and chronic intestinal inflammatory diseases in general (IBDs).

13. A medical device comprising a bacterial strain belonging to the *Lactobacillus kefiri* species, wherein said strain is *L. kefiri* SGL 13 deposited with accession number DSM 27331 on June 13, 2013.

## Patentansprüche

1. Bakterienstamm, der zur Spezies *Lactobacillus kefiri* gehört, wobei der Stamm L *kefiri* SGL 13 ist, der am 13. Juni 2013 unter der Hinterlegungsnummer DSM 27331 hinterlegt wurde.

2. Zusammensetzung, die den Bakterienstamm nach Anspruch 1 umfasst.

3. Zusammensetzung nach Anspruch 2, die ferner einen Pflanzenextrakt umfasst.

4. Zusammensetzung nach Anspruch 3, wobei der Pflanzenextrakt ein Extrakt von *Andrographis paniculata* ist.

5. Zusammensetzung nach einem der Ansprüche 2 bis 4, die ferner ein Präbiotikum umfasst.

6. Zusammensetzung nach einem der Ansprüche 2 bis 5 und pharmakologisch verträgliche Hilfsstoffe.

7. Zusammensetzung nach einem der Ansprüche 2 bis 6, wobei die Zusammensetzung probiotische Aktivität aufweist.

8. Zusammensetzung nach einem der Ansprüche 2 bis 7, wobei die Zusammensetzung in fester oder flüssiger Form vorliegt.

9. Zusammensetzung nach einem der Ansprüche 2 bis 8, wobei die Zusammensetzung eine Lebensmittelzusammensetzung ist.

10. Zusammensetzung nach einem der Ansprüche 2 bis 9 zur Verwendung als Medikament.

11. Zusammensetzung nach einem der Ansprüche 2 bis 10 zur Verwendung bei der Vorbeugung und Behandlung von Darmentzündungen und bei der Behandlung und Vorbeugung von Darmneoplasmen.

12. Zusammensetzung zur Verwendung nach Anspruch 11, wobei die Darmentzündungen ausgewählt sind aus der Gruppe, bestehend aus ischämischer Kolitis, lymphozytärer Kolitis, ulzerativer Kolitis, Diversionskolitis, Mangelerscheinungen, Morbus Crohn und Enterokolitis, wie nekrotisierender Enterokolitis, und allen akuten und chronisch-entzündlichen Darmerkrankungen im Allgemeinen (CED).

13. Medizinische Vorrichtung, die einen Bakterienstamm, der zur Spezies *Lactobacillus kefiri* gehört, umfasst, wobei der Stamm *L. kefiri* SGL 13 ist, der am 13. Juni 2013 unter der Hinterlegungsnummer DSM 27331 hinterlegt wurde.

## Revendications

1. Souche bactérienne appartenant à l'espèce *Lactobacillus kefiri,* où ladite souche est *L. kefiri* SGL 13 déposée sous le numéro d'accession DSM 27331 le 13 juin 2013.

2. Composition comprenant la souche bactérienne selon la revendication 1.

3. Composition selon la revendication 2, comprenant en outre un extrait végétal.

4. Composition selon la revendication 3, où ledit extrait végétal est un extrait *d'Andrographis paniculata.*

5. Composition selon l'une quelconque des revendications 2 à 4, comprenant en outre un prébiotique.

6. Composition selon l'une quelconque des revendications 2 à 5, et des excipients pharmacologiquement acceptables.

7. Composition selon l'une quelconque des revendications 2 à 6, où ladite composition a une activité probiotique.

8. Composition selon l'une quelconque des revendications 2 à 7, où ladite composition est sous une forme solide ou liquide.

9. Composition selon l'une quelconque des revendications 2 à 8, où ladite composition est une composition alimentaire.

10. Composition selon l'une quelconque des revendications 2 à 9, pour utilisation comme un médicament.

11. Composition selon l'une quelconque des revendications 2 à 10, destinée à être utilisée dans la prévention et le traitement des inflammations intestinales, et dans le traitement et la prévention des néoplasmes intestinaux.

12. Composition à utiliser selon la revendication 11, où lesdites inflammations intestinales sont choisies dans le groupe constitué par colite ischémique, colite lymphocytaire, colite ulcéreuse, colite de diversion, insuffisances, maladie de Chron et entérocolite, telle que l'entérocolite nécrosante, et toutes les maladies inflammatoires intestinales aiguës et chroniques en général (MICI).

13. Dispositif médical comprenant une souche bactérienne appartenant à l'espèce *Lactobacillus kefiri,* où ladite souche est *L. kefiri* SGL 13 déposée sous le numéro d'accession DSM 27331 le 13 juin 2013.
